# EUROPÄISCHE PATENTSCHRIFT

(11) **EP 0 668 283 B1**
(45) Veröffentlichungstag und Bekanntmachung des Hinweises auf die Patenterteilung: **20.05.1998**
(21) Anmeldenummer: 95101687.2
(22) Anmeldetag: 08.02.1995
(51) Int. Cl.: C07D 498/10, C07D 519/00, C08G 73/06

(54) **Verfahren zur Herstellung von Polyalkyl-1-oxadiazaspirodecan-Verbindungen**
Process for the preparation of polyalkyl-1-oxadiazaspirodecane compounds
Procédé pour la préparation des composés de polyalcoyl-1-oxadiazaspirodécane

(30) Priorität: 19.02.1994 DE 4405387
(43) Veröffentlichungstag der Anmeldung: 23.08.1995
(73) Patentinhaber: Clariant GmbH, 65929 Frankfurt am Main (DE)
(72) Erfinder: Gaa, Karl, Dr., D-89349 Burtenbach (DE); Nowy, Günter, Dr., D-86482 Aystetten (DE); Schmailzl, Georg, Dr., D-86356 Neusäss (DE)

(56) Entgegenhaltungen:
- EP-A- 0 224 181
- EP-A- 0 402 889

## Beschreibung

Die Erfindung bezieht sich auf ein Verfahren zur Herstellung von Polyalkyl-1-oxadiazaspirodecan-Verbindungen, die als hochwirksame Licht-schutzmittel für Polymere verwendet werden können.

Verbindungen der Formel 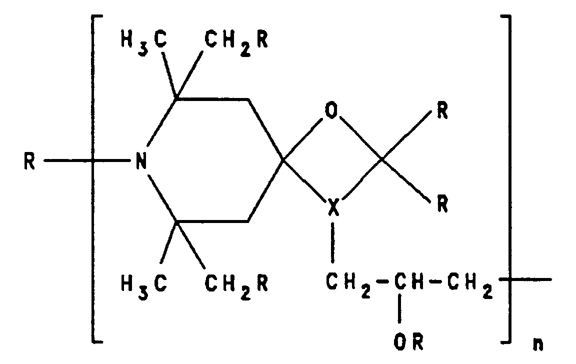 sind bekannt (vgl. EP 402 889). Das Herstellverfahren für diese Verbindungen ist dadurch gekennzeichnet, daß die Synthese in einem innerten Lösemittel in Anwesenheit von festem oder wässerigem Alkalimetallhydroxid und zusätzlich einem Phasentransferkatalysator erfolgt. Der Zusatz eines Phasentransferkatalysators hat jedoch den Nachteil, daß er bei der Aufarbeitung des Reaktionsgemisches in das Abwasser gelangt und somit eine Umweltbelastung darstellt. Insbesondere die als besonders wirksam beschriebenen quartären Ammonium- oder Phosphoniumhalogenide machen die Einleitung des Abwassers in eine biologische Kläranlage unmöglich, da quartäre Ammonium- und Phosphoniumsalze bakterizide Wirkung besitzen und nicht in einer biologischen Kläranlage aufgearbeitet werden können. Das Abwasser muß daher aufwendig als Sondermüll entsorgt werden.

Es bestand somit die Aufgabe, ein Verfahren zu finden, das die genannten Verbindungen bei möglichst kurzen Reaktionszeiten in möglichst hohen Ausbeuten bei mindestens gleicher Produktqualität möglich macht, ohne dabei die aus dem Stand der Technik bekannten Nachteile einer zu geringen Umweltverträglichkeit und einer dadurch bedingten aufwendigen Abwasserentsorgung aufzuweisen.

Es wurde gefunden, daß die Aufgabe gelöst werden kann, wenn man zur Herstellung der genannten Verbindungen als alleinigen Katalysator festes oder wässeriges Alkalimetallhydroxid verwendet und die Reaktion in einem Lösemittelgemisch bestehend aus mindestens einem C₁-C₄-Alkohol, ausgenommen Diethylenglykol und gegebenenfalls einem inerten Lösemittel, welches vorteilhaft wiedergewonnen werden kann, durchführt.

Die vorliegende Erfindung betrifft somit ein Verfahren zur Herstellung von Polyalkyl-1-oxa-diazaspirodecan-Verbindungen der Formel I 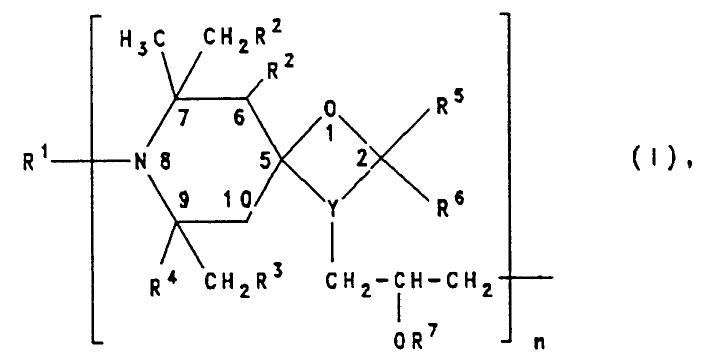 worin
- n: eine ganze Zahl von 1 bis 50 ist und
- Y: die Bedeutung einer Gruppe der Formel II oder III 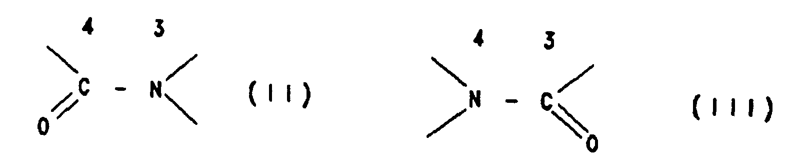
hat, wobei die Indices 3 bzw. 4 die Ringpositionen im Diazaspirodecansystem angeben und eine Bindung des Stickstoffs mit einer CH₂-Gruppe der Propylen-2-oxygruppe verknüpft ist,
R¹ ein Wasserstoffatom, ein Sauerstoffatom, eine NO-Gruppe, eine C₁-C₁₂--Alkylgruppe, eine Allylgruppe, eine C₁-C₂₂-Acylgruppe, eine Benzylgruppe, eine C₁-C₁₂-Alkyloxy-Gruppe oder eine C₃-C₁₂-Cycloalkoxy-Gruppe,
R² und R³ entweder gleich sind und ein Wasserstoffatom oder eine C₁-C₅-Alkylgruppe bedeuten, wobei dann R⁴ eine Methylgruppe ist, oder
R² ein Wasserstoffatom oder eine C₁-C₅-Alkylgruppe ist und R³ und R⁴ zusammen mit den sie verbindenden Kohlenstoffatomen eine C₅- oder C₆-Cycloalkylgruppe oder eine Gruppe der Formel 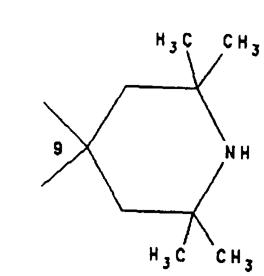 bilden,
R⁵ und R⁶ gleich oder verschieden sind und für ein Wasserstoffatom, eine C₁-C₃₀-,-Alkylgruppe, für eine unsubstituierte oder durch Chlor oder C₁-C₄-Alkyl substituierte C₇-C₁₂-Phenylalkylgruppe stehen, oder
R⁵ und R⁶ zusammen mit dem sie verbindenden Kohlenstoffatom eine unsubstituierte oder durch bis zu vier C₁-C₄-Alkylgruppen substituierte C₅-C₁₈-Cycloalkylgruppe oder eine Gruppe der Formel 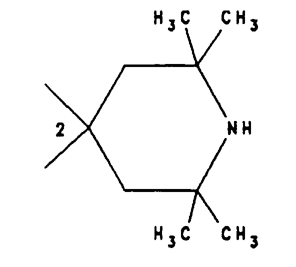 bilden,
R⁷ bei n = 1 keine Bedeutung hat, sodaß das Sauerstoffatom mit der endständigen CH₂-Gruppe verbunden ist und einen Oxiranring bildet,
R⁷ bei n > 1 ein Wasserstoffatom oder eine C₁-C₂₂-Acylgruppe ist, oder in der endständigen Monomereneinheit keine Bedeutung hat, so daß das Sauerstoffatom mit der enständigen CH₂-Gruppe verbunden ist und einen Oxiranring bildet, durch Umsetzung einer Verbindung der Formel IV 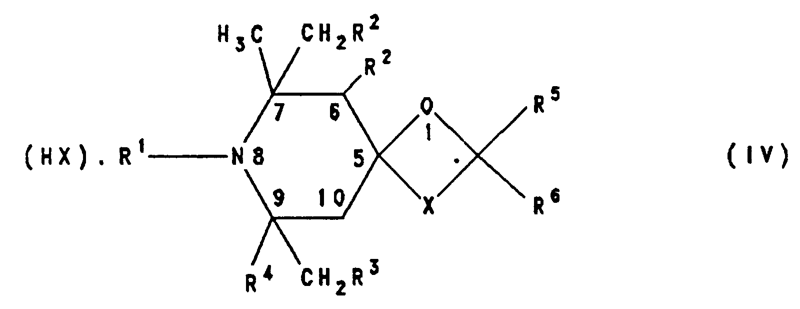 worin R¹, R², R³, R⁴, R⁵ und R⁶ die obengenannte Bedeutung haben und (HX) ein Säurerest ist, oder deren Salz mit einer Protonensäure mit einem Epihalohydrin der Formel V 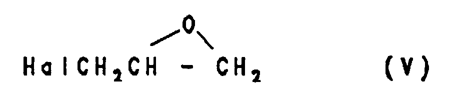 worin Hal ein Chlor-, Brom- oder Jodatom ist, im Molverhältnis 1:1 bis 1:10 in Anwesenheit eines Alkalihydroxyds in einem organischen Lösemittel und, bei n > 1, Erhitzen der entstandenen Epoxiverbindung VI 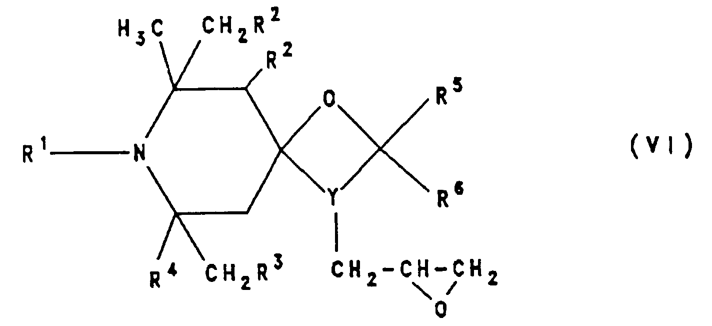 worin R¹, R², R³, R⁴, R⁵ und R⁶ die obengenannte Bedeutung haben, auf eine Temperatur von 100 bis 240°C, dadurch gekennzeichnet, daß die Umsetzung der Verbindung der Formel IV mit der Verbindung der Formel V in Gegenwart der äquimolaren bis zwanzigfach molaren Menge festen Alkalimetallhydroxids oder der entsprechenden Menge einer Mischung aus festem Alkalimetallhydroxid und Wasser im Gewichtsverhältnis 1:9 bis 9:1 als alleinigem Katalysator in einem Lösemittelgemisch aus mindestens einem C₁-C₄-Alkohol, ausgenommen Diethylenglykol, und gegebenenfalls einem inerten organischen Lösemittel durchgeführt wird.

In der Formel (I) der erfindungsgemäß herzustellenden Polyalkyl-1-oxa-diazaspirodecan-Verbindungen 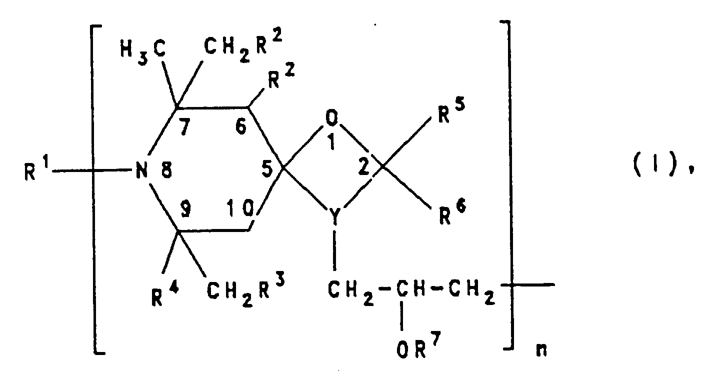 bedeutet
- n: eine ganze Zahl von 1 bis 50 , vorzugsweise 1 bis 15, insbesondere 1 bis 7.
- Y: hat die Bedeutung einer Gruppe der Formel II oder III 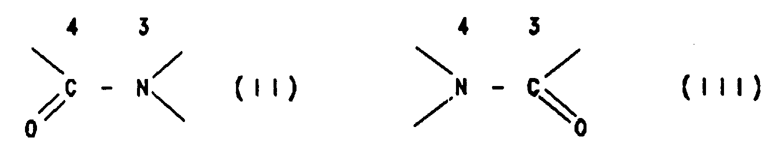 wobei die Indices 3 bzw. 4 die Ringpositionen im Diazaspirodecansystem angeben und eine Bindung des Stickstoffs mit einer CH₂-Gruppe der Propylen-2-oxygruppe verknüpft ist.

R¹ bedeutet ein Wasserstoffatom, ein Sauerstoffatom, eine NO-Gruppe, eine C₁-C₁₂-, vorzugsweise C₁-C₄-Alkylgruppe, eine Allylgruppe, eine C₁-C₂₂-Acylgruppe, vorzugsweise Acetylgruppe, eine Benzylgruppe, eine C₁-C₁₂-, vorzugsweise C₁-C₄-Alkyloxy-Gruppe oder eine C₃-C₁₂-, vorzugsweise C₃-C₆-Cycloalkoxy-Gruppe.

R² und R³ sind entweder gleich und bedeuten ein Wasserstoffatom oder eine C₁-C₅-Alkylgruppe, vorzugsweise ein Wasserstoffatom, wobei dann R⁴ eine Methylgruppe ist, oder
R² ist ein Wasserstoffatom oder eine C₁-C₅-Alkylgruppe und R³ und R⁴ bilden zusammen mit den sie verbindenden Kohlenstoffatomen eine C₅- oder C₆- Cycloalkylgruppe oder eine Gruppe der Formel 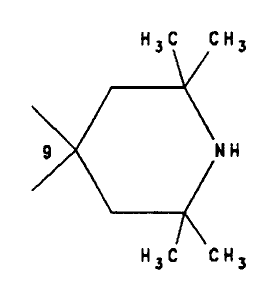 R⁵ und R⁶ sind gleich oder verschieden und stehen für ein Wasserstoffatom, eine C₁-C₃₀-, vorzugsweise C₁-C₁₈-Alkylgruppe, für eine unsubstituierte oder durch Chlor oder C₁-C₄-Alkyl substituierte C₇-C₁₂-Phenylalkylgruppe, oder
R⁵ und R⁶ bilden zusammen mit dem sie verbindenden Kohlenstoffatom eine unsubstituierte oder durch bis zu vier C₁-C₄-Alkylgruppen substituierte C₅-C₁₈-Cycloalkylgruppe oder eine Gruppe der Formel 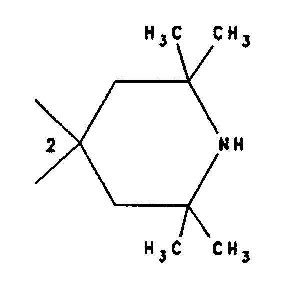 Bei n = 1 hat R⁷ keine Bedeutung, sodaß das Sauerstoffatom mit der endständigen CH₂-Gruppe verbunden ist und einen Oxiranring bildet.
Bei n > 1 ist R⁷ ein Wasserstoffatom oder eine C₁-C₂₂-, vorzugsweise C₁-C₁₂-Acylgruppe, oder hat in der endständigen Monomereneinheit keine Bedeutung, so daß das Sauerstoffatom mit der endständigen CH₂-Gruppe verbunden ist und einen Oxiranring bildet.

Die Herstellung der Verbindungen der Formel (I) erfolgt nach folgendem Reaktionsschema:

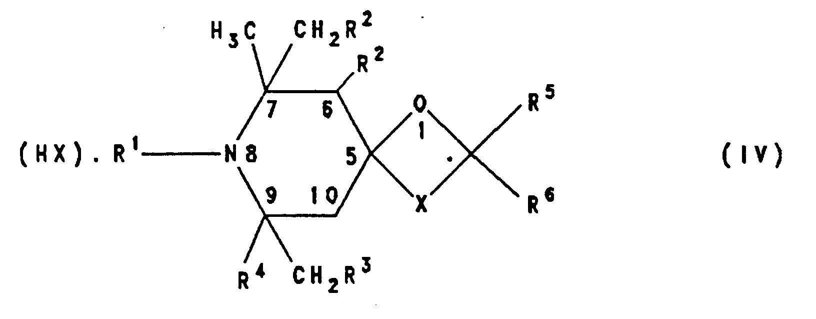

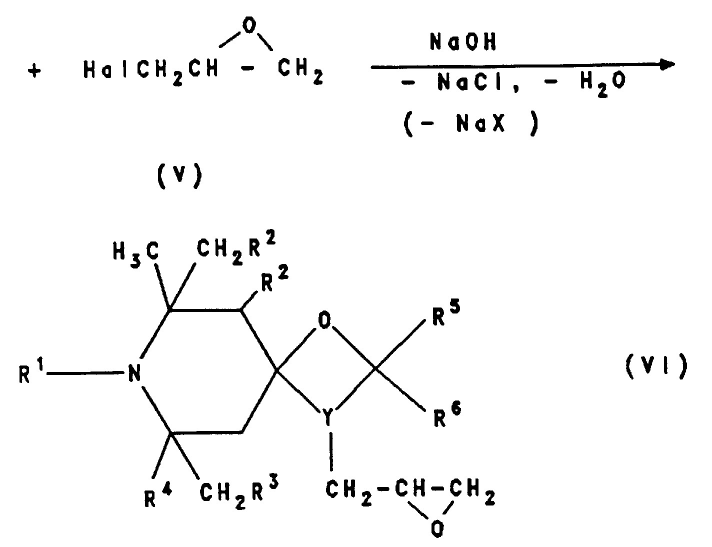

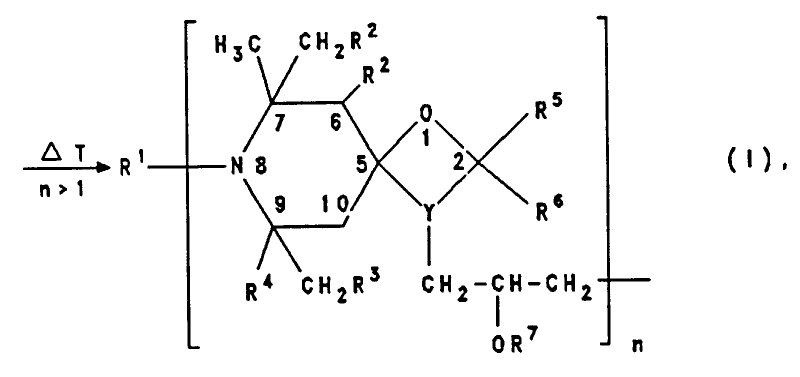

In den Formeln des Reaktionsschemas haben die Reste R², R³, R⁴, R⁵, R⁶, Y, Hal und n die oben angegebenen Bedeutungen: Der Rest R¹ bedeutet Wasserstoff und der Rest R⁷ bedeutet ebenfalls Wasserstoff oder hat in der endständigen Monomereneinheit keine Bedeutung, so daß das Sauerstoffatom mit der endständigen CH₂-Gruppe einen Oxiranring bildet.

Geeignete Verbindungen der Formel IV sind beispielsweise
2-iso-Propyl-7,7,9,9,-tetramethyl-1-oxa-3,8-diaza-4-oxo-spiro-[4,5]-decan,
2-Butyl-7,7,9,9-tetramethyl-1-oxa-3,8-diaza-4-oxo-spiro-[4,5]-decan,
2-iso-Butyl-7,7,9,9-tetramethyl-1-oxa-3,8-diaza-4-oxo-spiro-[4,5]-decan,
2-Pentyl-7,7,9,9-tetramethyl-1-oxa-3,8-diaza-4-oxo-spiro-[4,5]-decan,
2-Hexyl-7,7,9,9-tetramethyl-1-oxa-3,8-diaza-4-oxo-spiro-[4,5]-decan,
2-Heptyl-7,7,9,9-tetramethyl-1-oxa-3,8-diaza-4-oxo-spiro-[4,5]-decan,
2-iso-Heptyl-7,7,9,9-tetramethyl-1-oxa-3,8-diaza-4-oxo-spiro-[4,5]-decan,
2-iso-Octyl-7,7,9,9-tetramethyl-1-oxa-3,8-diaza-4-oxo-spiro-[4,5]-decan,
2-Nonyl-7,7,9,9-tetramethyl-1-oxa-3,8-diaza-4-oxo-spiro-[4,5]-decan,
2-iso-Nonyl-7,7,9,9-tetramethyl-1-oxa-3,8-diaza-4-oxo-spiro-[4,5]-decan,
2-Undecyl-7,7,9,9-tetramethyl-1-oxa-3,8-diaza-4-oxo-spiro-[4,5]-decan,
2-Phenyl-7,7,9,9-tetramethyl-1-oxa-3,8-diaza-4-oxo-spiro-[4,5]-decan,
2-(4-Chlor-phenyl)-7,7,9,9-tetramethyl-1-oxa-3,8-diaza-4-oxo-spiro-[4,5]-decan,
2,2-Dimethyl-7,7,9,9-tetramethyl-1-oxa-3,8-diaza-4-oxo-spiro-[4,5]-decan,
2-Ethyl-2,7,7,9,9-pentamethyl-1-oxa-3,8-diaza-4-oxo-spiro-[4,5]-decan,
2-Propyl-2,7,7,9,9-pentamethyl-1-oxa-3,8-diaza-4-oxo-spiro-[4,5]-decan,
2-iso-Propyl-6,7,7,9,9-pentamethyl-1-oxa-3,8-diaza-4-oxo-spiro-[4,5]-decan,
2-Butyl-2,7,7,9,9-pentamethyl-1-oxa-3,8-diaza-4-oxo-spiro-[4,5]-decan,
2-iso-Butyl-2,7,7,9,9-pentamethyl-1-oxa-3,8-diaza-4-oxo-spiro-[4,5]-decan,
2-Pentyl-6,7,7,9,9-pentamethyl-1-oxa-3,8-diaza-4-oxo-spiro-[4,5]-decan,
2-Hexyl-2,7,7,9,9-pentamethyl-1-oxa-3,8-diaza-4-oxo-spiro-[4,5]-decan,
2-Nonyl-2,7,7,9,9-pentamethyl-1-oxa-3,8-diaza-4-oxo-spiro-[4,5]-decan,
2,2-Diethyl-7,7,9,9-tetramethyl-1-oxa-3,8-diaza-4-oxo-spiro-[4,5]-decan,
2,2-Dipropyl-7,7,9,9-tetramethyl-1-oxa-3,8-diaza-4-oxo-spiro-[4,5]-decan,
2,2-Dibutyl-7,7,9,9-tetramethyl-1-oxa-3,8-diaza-4-oxo-spiro-[4,5]-decan,
2-Ethyl-2-Pentyl-7,7,9,9-tetramethyl-1-oxa-3,8-diaza-4-oxo-spiro-[4,5]-decan,
2,2-Dibenzyl-7,7,9,9-tetramethyl-1-oxa-3,8-diaza-4-oxo-spiro-[4,5]-decan,
2,2,4,4-Tetramethyl-7-oxa-3,12-diaza-14-oxo-dispiro-[5,1,4,2]-tetradecan,
2,2,4,4-Tetramethyl-7-oxa-3,20-diaza-21-oxo-dispiro-[5,1,11,2]-heneicosan,
2,2,4,4-Tetramethyl-7-oxa-3,14-diaza-15-oxo-dispiro-[5,5,5,2]-pentadecan,
2,2,4,4,10,10,12,12-Octamethyl-7-oxa-3,11,14-triaza-15-oxo-dispiro-[5,1,5,2]-pentadecan, pentadecan,
2-Ethyl-2,7,7,9,9-pentamethyl-1-oxa-3,8-diaza-4-oxo-8-oxyl-spiro-[4,5]-decan.

Die als Ausgangsprodukte verwendeten Polyalkyloxadiazaspirodecane sind bekannt und nach den in US 4 110 334 und US 4 107 139 angegebenen Vorschriften zugänglich.

Besonders bevorzugt unter den Verbindungen IV ist 2,2,4,4-Tetramethyl-7-oxa-3,20-diaza-21-oxo-dispiro-[5,1,11,2]-heneicosan oder dessen Hydrochlorid.

Die Verbindung der Formel IV wird mit einem Epihalogenhydrin der Formel V, worin unter Hal ein Chlor-, Brom- oder Jodatom, bevorzugt Chlor, zu verstehen ist, im Molverhältnis 1:1 bis 1:10, vorzugsweise 1:1 bis 1:5 und insbesondere 1:1 bis 1:3 umgesetzt. Die Reaktion erfolgt in einem Lösemittelgemisch bestehend aus mindestens einem Alkohol und gegebennenfalls einem inerten organischen Lösemittel in Anwesenheit der äquimolaren bis zwanzigfach molaren Menge festen Alkalimetallhydroxids oder der entsprechenden Menge einer Mischung aus festem Alkalimetall-hydroxid und Wasser im Gewichtsverhältnis 1:9 bis 9:1, vorzugsweise 4:6 bis 8:2, und insbesondere 5:5 bis 7:3.

Die Reaktionstemperatur liegt im Bereich von 20 bis 220°C, vorzugsweise 40 bis 120°C und insbesondere 60 bis 100°C.

Als organisches Lösemittel enthält der Reaktionsansatz mindestens einen Alkohol. Als Alkohol eignet sich ein geradkettiger und verzweigter Alkohol mit Kettenlänge C₁ bis C₄ ausgenommen Diethylenglykol und insbesondere Isopropanol. Der Alkohol wird in einer Menge von 1 bis 100, vorzugsweise 20 bis 80, insbesondere 30 bis 70 Gew.-%, bezogen auf die gesamte Lösemittelmenge, eingesetzt.

Neben dem Alkohol enthält der Reaktionsansatz ein inertes organisches Lösemittel. Als solches kommt ein aliphatischer oder aromatischer Kohlenwasserstoff, wie beispielsweise Petrolether, Hexan, Heptan, Benzinschnitte, Toluol, Cyclohexan, Xylol etc. in Frage.

Die Reaktion ist im allgemeinen nach 30 bis 60 Minuten beendet.

Nach der Reaktion wird das Reaktionsgemisch soweit eingeengt, bis der Alkohol und das überschüssige Epihalohydrin vollständig abdestilliert und somit zugleich zurückgewonnen sind.

Danach wird frisches inertes Lösemittel und Wasser zu der Reaktionsmischung gegeben. Die Phasen werden getrennt, die organische Phase wird mehrfach mit Wasser gewaschen und das Lösemittel abdestilliert, wobei das Produkt zugleich azeotrop getrocknet wird.

Das dabei meist ölig anfallende Epoxid VI läßt sich isolieren (wenn n = 1) oder ohne weitere Reinigung durch Erhitzen auf 100 bis 240°C, vorzugsweise 120 bis 220°C und insbesondere 150 bis 200°C in ein festes, amorphes, zunächst glasartig anfallendes Polymer I mit n > 1 überführen. Niedrige Polymerisationsgrade können durch kurze und hohe Polymerisationsgrade durch lange Polymerisierdauer erzielt werden. Weiterhin besteht bei gleicher Polymerisationsdauer mit steigender Temperatur die Tendenz zu höheren Polymerisationsgraden. Bei unter gleichen Polymerisationsbedingungen erhaltenen Produkten ist die Lösungsviskosität abhängig vom Grad der Umsetzung von Verbindung IV mit dem Epihalohydrin V und ist somit ein Maß für die Reinheit des Epoxids VI vor der Polymerisation.

Nach der Polymerisation kann das Polymer - wenn gewünscht - an den Positionen R¹ und R⁷ des Moleküls nach an sich bekannten Methoden derivatisiert werden.

Die nach erfindungsgemäßem Verfahren hergestellten Verbindungen dienen als Lichtstabilisatoren in organischen Polymeren, beispielsweise in den nachstehend aufgeführten:
1. Polymere von Mono- und Diolefinen, beispielsweise Polyethylen hoher, mittlerer oder niederer Dichte (das gegebenenfalls vernetzt sein kann), Polypropylen, Polyisobutylen, Polybuten-1, Polymethylpenten-1, Polyisopren oder Polybutadien sowie Polymerisate von Cycloolefinen, wie beispielsweise von Cyclopenten oder Norbornen.
2. Mischungen der unter 1) genannten Polymeren, beispielsweise Mischungen von Polypropylen mit Polyethylen oder mit Polyisobutylen.
3. Copolymere von Mono- und Diolefinen untereinander oder mit anderen Vinylmonomeren, wie beispielsweise Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.
4. Polystyrol, Poly(p-methylstyrol).
5. Copolymere von Styrol oder α-Methylstyrol mit Dienen oder Acrylderivaten, wie beispielsweise Styrol-Butadien, Styrol-Maleinsäureanhydrid, Styrol-Acrylnitril, Styrol-Ethylmethacrylat, Styrol-Butadien-Ethylacrylat, Styrol-Acrylnitril-Methacrylat; Mischungen von hoher Schlagzähigkeit aus Styrol-Copolymeren und einem anderen Polymer, wie beispielsweise einem Polyacrylat, einem Dien-Polymeren oder einem Ethylen-Propylen-Dien-Terpolymeren; sowie Block-Copolymere des Styrols, wie beispielsweise Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol, wie beispielsweise Styrol auf Polybutadien, Styrol und Acrylnitril auf Polybutadien, Styrol und Maleinsäureanhydrid auf Polybutadien, Styrol und Alkylacrylate oder Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, die beispielsweise als sogenannte ABS-, MBS-, ASA- oder AES-Polymere bekannt sind.
7. Polyvinylchlorid.
8. Mischpolymerisate des Vinylchlorids, welche durch die bekannten Verfahren hergestellt werden können (beispielsweise Suspensions-, Masse- oder Emulsionspolymerisation).
9. Mischpolymere des Vinylchlorids mit bis zu 30 Gew.-% an Comonomeren, wie beispielsweise Vinylacetat, Vinylidenchlorid, Vinylether, Acrylnitril, Acrylsäureester, Maleinsäuremono- oder -diester oder Olefinen, sowie Pfropfpolymerisate des Vinylchlorids.
10. Halogenhaltige Polymere, wie beispielsweise Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie beispielsweise, Polyvinylidenchlorid, Polyvinylfluorid, Polyvinylidenfluorid; sowie deren Copolymere wie von Vinylchlorid-Vinylidenchlorid, Vinylchlorid-Vinylacetat oder Vinylidenchlorid-Vinylacetat.
11. Polymere, die sich von α-β-ungesättigten Säuren und deren Derivaten ableiten, wie Polyacrylate und Polymethacrylate, Polyacrylamide und Polyacrylnitrile.
12. Copolymere der unter 11) genannten Monomeren untereinander oder mit anderen ungesättigten Monomeren, wie beispielsweise Acrylnitril-Butadien-Copolymere, Acrylnitril-Alkylacrylat-Copolymere, Acrylnitril-Alkoxyacrylat-Copolymere, Acrylnitril-Vinylhalogenid-Copolymere oder Acrylnitril-Alkylmethacrylat-Butadien-Copolymere.
13. Polymere, die sich von ungesättigten Alkoholen und Aminen bzw. deren Acylderivaten oder Acetalen ableiten, wie Polyvinylalkohol, Polyvinylacetat,-stearat, -benzoat, -maleat, Polyvinylbutyral, Polyallylphthalat, Polyallylmelamin.
14. Homo- und Copolymere von cyclischen Ethern, wie Polyethylenglykole, Polyethylenoxyd, Polypropylenoxyd oder deren Copolymere mit Bisglycidylethern.
15. Polyacetale, wie Polyoxymethylen, sowie solche Polyoxymethylene, die Comonomere wie beispielsweise Ethylenoxyd enthalten.
16. Polyphenylenoxyde und -sulfide und deren Mischungen mit Styrolpolymeren.
17. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte (Polyisocyanate-Polyole-Prepolymere).
18. Polyamide und Copolyamide, die sich von Diaminen und Dicarbonsäuren und/oder von Aminocarbonsäuren oder den entsprechenden Lactamen ableiten, wie Polyamid-4, Polyamid-6, Polyamid-6,6, Polyamid-6.10, Polyamid-11, Polyamid-12, Poly-2,4,4-trimethylhexamethylenterephthalamid, Poly-m-phenylenisophthalamid, sowie deren Copolymere mit Polyethern, wie beispielsweise mit Polyethylenglykol, Polypropylenglykol oder Polytetramethylenglykol.
19. Polyharnstoffe, Polyimide und Polyamid-imide.
20. Polyester, die sich von Dicarbonsäuren und Diolen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Poly-(2,2-bis-(4-hydroxyphenyl)-propan)-terephthalat, Polyhydroxybenzoate, sowie Block-Polyether-ester, die sich von Polyethylen mit Hydroxyendgruppen, Dialkoholen und Dicarbonsäuren ableiten.
21. Polycarbonate und Polyestercarbonate.
22. Polysulfone, Polyethersulfone und Polyetherketone.
23. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
24. Trocknende und nicht trocknende Alkydharze.
25. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
26. Vernetzbare Acrylharze, die sich von substituierten Acrylsäureestern ableiten, wie beispielsweise Epoxyacrylaten, Urethan-acrylaten oder Polyesteracrylaten.
27. Alkydharze, Polyesterharze und Acrylatharze, die mit Melaminharzen, Harnstoffharzen, Polyisocyanaten oder Epoxidharzen vernetzt sind.
28. Vernetzbare Epoxidharze, die sich von Polyepoxiden ableiten, beispielsweise von Bis-glycidylethern oder von cycloaliphatischen Diepoxiden.
29. Natürliche Polymere, wie Cellulose, Naturkautschuk, Gelatine sowie deren polymerhomolog chemisch abgewandelte Derivate, wie Celluloseacetate, -propionate und -butyrate, oder Celluloseether, wie Methylcellulose.
30. Mischungen der oben erwähnten Polymeren, wie beispielsweise PP/EPDM, Polyamid-6/EPDM oder ABS, PVC/EVA, PVC/ABS, PVC/MBS, PC/ABS, PBTP/ABS, PC/ASA, PC/PBT, PVC/CPE, PVD/Acrylat, POM/thermoplastisches PUR, POM/Acrylat, POM/MBS, PPE/HIPS, PPE/Polyamid-6.6 und Copolymere, PA/HDPE, PA/PP, PA/PPE.
31. Natürlich vorkommende und synthetische organische Stoffe, welche reine Monomere oder Mischungen von Monomeren sind, wie beispielsweise Mineralöle, tierische und pflanzliche Fette, Öle und Wachse, oder Öle, Fette und Wachse auf Basis synthetischer Ester oder Mischungen dieser Stoffe.
32. Wäßrige Dispersionen von Natur- oder Synthesekautschuk.

Die zu stabilisierenden organischen Polymeren können noch weitere Zusatzstoffe enthalten, beispielsweise noch folgende Antioxidantien:
1.Alkylierte Monophenole, beispielsweise
   2,6-Di-t-butyl-4-methylphenol, 2-t-Butyl-4,6-dimethylphenol, 2,6-Di-t-butyl-4-ethylphenol, 2,6-Di-t-butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-t-butyl-4-methoxymethylphenol.
2.Alkylierte Hydrochinone, beispielsweise
   2,6-Di-t-butyl-4-methoxyphenol, 2,5-Di-t-butyl-hydrochinon, 2,5-Di-t-amylhydrochinon, 2,6-Diphenyl-4-octadecyloxyphenol.
3.Hydroxylierte Thiodiphenylether, beispielsweise
   2,2'-Thio-bis-(6-t-butyl-4-methylphenol), 2,2'-Thio-bis-(4-octylphenol), 4,4'-Thio-bis-(6-t-butyl-3-methylphenol), 4,4'-Thio-bis-(6-t-butyl-2-methylphenol).
4.Alkyliden-Bisphenole, beispielsweise
   2,2'-Methylen-bis-(6-t-butyl-4-methylphenol), 2,2'-Methylen-bis(6-t-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-t-butylphenol), 2,2'-Ethyliden-bis-(4,6-di-t-butylphenol), 2,2'-Ethyliden-bis-(6-t-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-t-butylphenol), 4,4'-Methylen-bis(6-t-butyl-2-methylphenol), 1,1-Bis-(5-t-butyl-4-hydroxy-2-methylphenyl)-butan, 2,6-Di-(3-t-butyl-5-methyl-2-hydroxybenzyl)-4-methylphenol, 1,1,3-Tris-(5-t-butyl-4-hydroxy-2-methylphenyl)-butan, 1,1-Bis-(5-t-butyl-4-hydroxy-2-methylphenyl)-3-n-dodecylmercaptobutan, Di-(3-t-butyl-4-hydroxy-5-methylphenyl)-dicyclo-pentadien, Di-[2-(3'-t-butyl-2'-hydroxy-5'-methyl-benzyl)-6-t-butyl-4-methyl-phenyl]-terephthalat,
   Ethylenglykol-bis-[3,3-bis-(3'-t-butyl-4'-hydroxyphenyl)-butyrat].
5.Benzylverbindungen, beispielsweise
   1,3,5-Tri-(3,5-di-t-butyl-4-hydroxybenzyl)-2,4, 6-trimethylbenzol, Di-(3,5-di-t-butyl-4-hydroxybenzyl)-sulfid, 3,5-Di-t-butyl-4-hydroxybenzyl-mercaptoessigsäure-isooctylester, Bis- (4-t-butyl-3-hydroxy-2, 6-d imethylbenzyl)-dithiol-terephthalat, 1,3, 5-Tris-(3,5-di-t-butyl-4-hydroxybenzyl)-isocyanurat, 1,3,5-Tris-(4-t-butyl-3-hydroxy-2,6-dimethylbenzyl)-isocyanurat, 3,5-Di-t-butyl-4-hydroxybenzylphosphonsäure-dioctadecylester, Calciumsalz des 3,5-Di-t-butyl-4-hydroxybenzylphosphonsäure-mono-ethylesters.
6.Acylaminophenole, beispielsweise
   4-Hydroxy-laurinsäureanilid, 4-Hydroxy-stearinsäureanilid, 2,4-Bis-octylmercapto-6-(3, 5-di-t-butyl-4-hydroxy-anilino)-s-triazin, N-(3, 5-Di-t-butyl-4-hydroxyphenyl)-carbaminsäure-octylester.
7.Ester der β-(3,5-Di-t-butyl-4-hydroxyphenyl)-propionsäure mit ein- oder mehrwertigen Alkoholen, wie z.B. mit
   Methanol, Diethylenglykol, Octadecanol, Triethylenglykol, 1,6-Hexandiol, Pentaerythrit, Neopentylglykol, Tris-hydroxyethyl-isocyanurat, Thiodiethylenglykol, Di-hydroxyethyl-oxalsäurediamid.
8.Ester der β-(5-t-Butyl-4-hydroxy-3-methylphenyl)-propionsäure mit ein- oder mehrwertigen Alkoholen, wie z.B. mit
   Methanol, Diethylenglykol, Octadecanol, Triethylenglykol, 1,6-Hexandiol, Pentaerythrit, Neopentylglykol, Tris-hydroxyethyl-isocyanurat, Thiodiethylenglykol, Di-hydroxyethyl-oxalsäurediamid.
9.Amide der β-(3,5-Di-t-butyl-4-hydroxyphenyl)-propionsäure, wie z.B.
   N,N'-Di-(3,5-di-t-butyl-4-hydroxyphenylpropionyl)-hexamethylendiamin, N,N'-Di-(3,5-di-t-butyl-4-hydroxyphenylpropionyl)-trimethylendiamin, N,N'-Di-(3,5-di-t-butyl-4-hydroxyphenylpropionyl)-hydrazin.

Daneben können die zu stabilisierenden Polymeren noch weitere Additive enthalten, wie beispielsweise:
1.UV-Absorber und Lichtschutzmittel
   1.1 2-(2'-Hydroxyphenyl)-benztriazole, wie z.B. das 5'-Methyl-, 3',5'-Di-t-butyl, 5'-t-Butyl-, 5'-(1,1,3,3-Tetramethylbutyl)-, 5-Chlor-3',5'-di-t-butyl, 5-Chlor-3'-t-butyl-5'-methyl-, 3'-sec.-Butyl-5'-t-butyl-, 4'-Octoxy-, 3',5'-Di-t-amyl-, 3',5'-Bis(α,α-dimethylbenzyl)-Derivat.
   1.2 2-Hydroxybenzophenone, beispielsweise das 4-Hydroxy-, 4-Methoxy, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   1.3 Ester von gegebenenfalls substituierten Benzoesäuren, beispielsweise
      4-t-Butyl-phenylsalicylat, Phenylsalicylat, Octylphenylsalicyalat, Dibenzoylresorcin, Bis-(4-t-Bis-(4-t-butylbenzoyl)resorcin, Benzoylresorcin, 3,5-Di-t-butyl-4-hydroxybenzoesäure-2,4-di-t-butylphenylester, 3,5-Di-t-butyl-4-hydroxybenzoesäure-hexadecylester.
   1.4 Acrylate, beispielsweise
      α-Cyan-β,β-diphenylacrylsäure-ethylester bzw. -iso-octylester, α-Carbomethoxyzimtsäuremethylester, α-Cyano-β-methyl-p-methoxyzimtsäuremethylester bzw. -butylester, α-Carbomethoxy-p-methoxyzimtsäure-methylester, N-(β-Carbomethoxy-9-cyano-vinyl)-2-methyl-indolin.
   1.5 Nickelverbindungen, beispielsweise
      Nickelkomplexe des 2,2'-Thio-bis-[4-(1,1,3,3-tetramethyl-butyl)-phenols], wie der 1:1- oder 1:2-Komplex, gegebenenfalls mit zusätzlichen Liganden wie n-Butyl-amin, Triethanolamin oder N-Cyclohexyl-diethanolamin, Nickelalkyl-dithiocarbamate, Nickelsalze von 4-Hydroxy-3,5-di-t-butyl-benzylphosphonsäure-mono-alkylestern wie vom Methyl- oder Ethylester, Nickelkomplexe von Ketoximen wie vom 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden, Nickelsalze der 2-Hydroxy-4-alkoxybenzophenone.
   1.6 Sterisch gehinderte Amine, beispielsweise
      1.6.1 Bis(2,2,6,6-tetramethylpiperidyl)-sebacat, Bis-(1,2,2,6,6-pentamethylpiperidyl)-sebacat, Bis-(2,2,6,6-tetramethylpiperidyl)-glutarat, Bis-(1,2,2,6,6-pentamethylpiperidyl)-glutarat, Bis-(2,2,6,6-tetramethylpiperidyl)-succinat,
         Bis-(1,2,2,6,6-pentamethylpiperidyl)-succinat, 4-Stearyloxy-2,2,6,6-tetramethylpiperidin, 4-Stearyloxy-1,2,2,6,6-pentamethylpiperidin, 4-Stearoyloxy-2,2,6,6-tetramethylpiperidin, 4-Stearoyloxy-1,2,2,6,6-pentamethylpiperidin, 2,2,6,6-Tetramethylpiperidylbehenat, 1,2,2,6,6-Pentamethylpiperidylbehenat, 2, 2,4,4-Tetramethyl-7-oxa-3, 20-diazadispiro-[5.1.11.2]-heneicosan-21-on, 2,2,3,4,4-Penta-methyl-7-oxa-3,20-diazadispiro-[5.1.11.2]-heneicosan-21-on, 2,2,4,4-Tetramethyl-3-acetyl-7-oxy-3,20-diaza-dispiro-[5.1.11.2]-heneicosan-21-on,
         2,2,4,4-Tetramethyl-7-oxa-3,20-diaza-20-(β-lauryloxy-carbonylethyl)-21-oxodispiro-[5.1.11.2]-heneicosan, 2,2,3,4,4-Pentamethyl-7-oxa-3,20-diaza-20-(β-lauryloxy-carbonylethyl)-21-oxo-dispiro-[5.1.11.2]-heneicosan, 2,2,4,4-Tetramethyl-3-acetyl-7-oxa-3,20-diazo-20-(β-lauryloxycarbonyl-ethyl)-21-oxodispiro-[5.1.11.2]-heneicosan,
         1,1',3,3',5,5'-Hexahydro-2,2',4,4',6,6'-hexaaza-2,2',6,6'-bismethano-7,8-dioxo-4,4'-bis-(1,2,2,6,6-pentamethyl-4-piperidyl)biphenyl, N,N',N",N"'-Tetrakis-[2,4-bis-[N-(2,2,6,6-tetramethyl-4-piperidyl)-butylamino]-1,3,5-triazin-6-yl]-4,7-diazadecan-1,10-diamin, N,N',N",N"'-Tetrakis[2,4-bis-[N(1,2,2,6,6-pentamethyl-4-piperidyl)-butylamino]-1,3,5-triazin-6-yl]-4,7-diazadecan-1,10-diamin, N,N',N",N"'-Tetrakis-[2,4-bis-[N-(2,2,6,6-tetramethyl-4-piperidyl)-methoxypropylamino]-1,3,5-triazin-6-yl]-4,7-diazadecan-1,10-diamin, N,N',N",N"'-Tetrakis-[2,4-bis-[N-(1,2,2,6,6-pentamethyl-4-piperidyl)-methoxypropylamino]-1,3, 5-triazin-6-yl]-4,7-diazadecan-1,10-diamin,
         Bis-(1,2,2,6,6-pentamethyl-piperidyl)-n-butyl-3,5-di-t-butyl-4-hydroxybenzylmalonat,
         Tris-(2,2,6, 6-tetramethyl-4-piperidyl)-nitrilotriacetat, Tetrakis-(2,2,6,6-tetramethyl-4-piperidyl)-1,2,3,4-butantetracarbonsäure, 1,1'-(1,2-Ethandiyl)-bis-(3,3,5,5-tetramethyl-piperazinon).
      1.6.2 Poly-N,N'-bis(2,2,6, 6-tetramethyl-4-piperidyl)-1,8-diazadecylen, Kondensationsprodukt aus 1 -(2-Hydroxyethyl)-2,2,6,6-tetramethyl-4-hydroxypiperidin und Bernsteinsäure, Kondensationsprodukt aus N,N'-Bis-(2,2,6,6-tetramethyl-4-piperidyl)-hexamethylendiamin und 4-t-Octylamino-2,6-dichlor-1,3,5-triazin, Kondensationsprodukt aus N,N'-Bis-(2,2,6,6-tetramethyl-4-piperidyl)-hexamethylendiamin und 4-Morpholino-2,6-dichlor-1,3,5-triazin.
   1.7 Oxalsäurediamide, beispielsweise
      4,4'-Di-octyloxy-oxanilid, 2,2'-Di-octyloxy-5, 5'-di-t-butyl-oxanilid, 2,2'-Didodecyloxy-5, 5'-di-t-butyloxanilid, 2-Ethoxy-2'-ethyl-oxanilid, N,N'-Bis-(3-dimethylaminopropyl)-oxalamid, 2-Ethoxy-5-t-butyl-2'-ethyloxanilid und dessen Gemisch mit 2-Ethoxy-2'-ethyl-5,4-di-t-butyl-oxanilid, Gemische von o- und p-Methoxy- sowie von o- und p-Ethoxy-di-substituierten Oxaniliden.
   2. Metalldesaktivatoren, beispielsweise
      N,N'-Diphenyloxalsäurediamid, N-Salicylyl-N'-salicyloyl-hydrazin, N,N'-Bis-salicyloylhydrazin, N,N'-Bis-(3,5-di-t-butyl-4-hydroxyphenylpropionyl)-hydrazin, 3-Salicyloylamino-1,2,3-triazol, Bis-benzyliden-oxalsäuredihydrazid.
   3. Phosphite und Phosphonite, beispielsweise
      Triphenylphosphit, Diphenylalkylphosphite, Phenyldialkylphosphite, Trisnonylphenylphosphit, Trilaurylphosphit, Trioctadecylphosphit, Distearylpentaerythrityldiphosphit, Tris-(2,4-di-t-butylphenyl)-phosphit, Diisodecyl-pentaerythrityldiphosphit, Bis-(2,4-di-t-butylphenyl)-pentaerythrityl-diphosphit, Tristearylsorbityltriphosphit, Tetrakis-(2,4-di-t-butylphenyl)-4,4'-biphenylen-diphosphonit, 3,9-Bis-(2,4-di-t-butylphenoxy)-2,4,8,10-tetraoxa-3,9-diphosphaspiro-[5.5]-undecan, Tris-(2-t-butyl-4-thio-(2'-methenyl-4'-hydroxy-5'-t-butyl)-phenyl-5-methenyl)-phenylphosphit.
   4. Peroxidzerstörende Verbindungen, beispielsweise
      Ester der β-Thio-dipropionsäure, wie beispielsweise der Lauryl-, Stearyl-, Myristyl- oder Tridecylester, Mercaptobenzimidazol, das Zinksalz des 2-Mercaptobenzimidazols, Zink-alkyl-dithiocarbamate, Dioctadecylsulfid, Dioctadecyldisulfid, Pentaerythrit-tetrakis-(β-dodecylmercapto)-propionat.
   5. Basische Co-Stabilisatoren, beispielsweise
      Melamin, Polyvinylpyrrolidon, Dicyandiamid, Triallylcyanurat, Harnstoff-Derivate, Hydrazin-Derivate, Amine, Polyamine, Polyurethane, Alkali- und Erdalkalisalze höherer Fettsäuren oder Phenolate, beispielsweise Ca-Stearat, Zn-Stearat, Mg-Stearat, Na-Ricinoleat, K-Palmitat, Antimonbrenzcatechinat oder Zinnbrenzcatechinat, Hydroxide und Oxide von Erdalkalimetallen oder des Aluminiums, beispielsweise CaO, MgO, ZnO.
   6. Nukleierungsmittel, beispielsweise
      4-t-Butylbenzoesäure, Adipinsäure, Diphenylessigsäure, Dibenzylidensorbitol.
   7. Füllstoffe und Verstärkungsmittel, beispielsweise
      Calciumcarbonat, Silikate, Glasfasern, Asbest, Talk, Kaolin, Glimmer, Bariumsulfat, Metalloxide und -hydroxide, Ruß, Graphit.
   8. Sonstige Zusätze, beispielsweise
      Weichmacher, Gleitmittel, Emulgatoren, Pigmente, optische Aufheller, Flammschutzmittel, Antistatika, Treibmittel.

Die verschiedenen zusätzlichen Additive der vorgenannten Gruppen 1 bis 6 werden den zu stabilisierenden Polymeren in einer Menge von 0,01 bis 10, vorzugsweise 0,01 bis 5 Gew.-%, bezogen auf das Gesamtgewicht der Formmasse, zugesetzt. Der Mengenanteil der Additive der Gruppen 7 und 8 beträgt 1 bis 80, vorzugsweise 10 bis 50 Gew.-%, bezogen auf die gesamte Formmasse.

Die Additive werden nach allgemein üblichen Methoden in die organischen Polymeren eingearbeitet. Die Einarbeitung kann beispielsweise durch Einmischen oder Aufbringen der Verbindungen und gegebenenfalls weiterer Additive in oder auf das Polymere unmittelbar nach der Polymerisation oder in die Schmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglichem Verdunstenlassen des Lösemittels kann die Einarbeitung erfolgen. Die Verbindungen sind auch wirksam, wenn sie in ein bereits granuliertes Polymer nachträglich in einem gesonderten Verarbeitungsschritt eingebracht werden.

Die erfindungsgemäß hergestellten Verbindungen können auch in Form eines Masterbatches, der diese Verbindungen beispielsweise in einer Konzentration von 1 bis 75, vorzugsweise 2,5 bis 30 Gew.-% enthält, den zu stabilisierenden Polymeren zugesetzt werden.

Das erfindungsgemäße Verfahren bietet den Vorteil, daß durch Verwendung eines Lösemittelgemisches unter Verzicht auf einen Phasentransferkatalysator höhere Umsetzungsgrade und somit höhere Produktqualität bei gleichbleibender Ausbeute erreicht werden. Überraschenderweise wird dabei unter den stark alkalischen Bedingungen keine Reaktion des Epichlorhydrins mit dem im Lösemittelgemisch enthaltenen beobachtet, wie es an sich zu erwarten wäre. Der eingesetzte Alkohol konnze in allen Fällen vollständig wiedergewonnen werden.

Die nachfolgenden Beispiele und Vergleichsbeispiele dienen der Erläuterung des Erfindungsgegenstandes.

### Beispiel 1 bis 6

### 2,2,4,4-Tetramethyl-7-oxa-3,20-diaza-20-(2,3-epoxypropyl)-21-oxo-dispiro-[5,1,11,2]-heneicosan und daraus erhaltenes Oligomer.

Zu 180 g eines Gemisches aus Xylol und Isopropanol im in Tabelle 1 angegebenen Verhältnis wurden nacheinander 100,0 g (0,25 mol) 2,2,4,4-Tetramethyl-7-oxa-3,20-diaza-21-oxo-dispiro-[5,1,11,2]-heneicosan-Hydrochlorid sowie 69,4 g (0,75 mol) Epichlorhydrin und 72,8 g 50%ige wässrige Natronlauge (0,91 mol NaOH) gegeben. Diese Mischung wurde 30 Minuten bei 80°C gerührt. Der Ansatz wurde im Vakuum eingeengt, bis alles Isopropanol und Epichlorhydrin abdestilliert waren. Das Destillat kann für weitere Ansätze verwendet werden. Der Reaktionsmischung wurden 110 g Xylol und 110 g Wasser zugesetzt und die Phasen getrennt. Die organische Phase wurde noch zweimal mit je 70 g Wasser gewaschen. Nach dem Abdestillieren des Lösemittels im Vakuum erhielt man ein farbloses Öl, welches die in der Überschrift angegebene Epoxyverbindung war. Diese wurde bei 200°C im Vakuum drei Stunden polymerisiert. Man erhielt ein sprödes, farbloses Harz, dessen Ausbeute und Lösungsviskosität ebenfalls in Tabelle 1 zusammengefaßt sind.

**Tabelle 1**

| Beisp. | Xylol/Iso¹) | Ausbeute [%] | Visk.²) [mm²/s] |
|---|---|---|---|
| 1 | 8/1 | 97,0 | 1,69 |
| 2 | 7/2 | 96,3 | 1,81 |
| 3 | 2/1 | 97,0 | 1,97 |
| 4 | 5/4 | 97,2 | 2,04 |
| 5 | 1/2 | 97,8 | 2,12 |
| 6 | 0/1 | 98,1 | 2,01 |

| | | | |
|---|---|---|---|
| ¹) Isopropanol | | | |
| ²) 20%ige Lösung in Toluol bei 25°C nach DGF-M-III 8(75) | | | |

### Vergleichsbeispiele A und B

2,2,4,4-Tetramethyl-7-oxa-3,20-diaza-20-(2,3-epoxypropyl)-21-oxo-dispiro-[5,1,11,2]-heneicosan und daraus erhaltenes Oligomer.

Zu 180 g Xylol wurden nacheinander 100,0 g (0,25 mol) 2,2,4,4-Tetramethyl-7-oxa-3,20-diaza-21-oxo-dispiro-[5,1,11,2]-heneicosan-Hydrochlorid, 1,3 g Polyethylenglykol 200 (Vergleichsbeispiel A) bzw. 10 Tropfen Tricaprylammoniumchlorid (Vergleichsbeispiel B) als Phasentransferkatalysator sowie 69,4 g (0,75 mol) Epichlorhydrin und 72,8 g 50%ige wässrige Natronlauge (0,91 mol NaOH) gegeben. Diese Mischung wurde 30 Minuten bei 80°C gerührt. Nach dem Abdestillieren des überschüssigen Epichlorhydrins wurden der Reaktionsmischung 110 g Xylol und 110 g Wasser zugesetzt und die Phasen getrennt. Die organische Phase wurde noch zweimal mit je 70 g Wasser gewaschen. Nach dem Abdestillieren des Lösemittels im Vakuum erhielt man ein farbloses Öl, welches die in der Überschrift angegebene Epoxyverbindung war. Diese wurde bei 200°C im Vakuum drei Stunden polymerisiert. Man erhielt ein sprödes, farbloses Harz dessen Ausbeute und Lösungsviskosität in Tabelle 2 zusammengefaßt sind.

**Tabelle 2**

| Vergl.bsp. | Ausbeute [%] | Visk.¹) [mm²/sec] |
|---|---|---|
| A | 95,6 | 1,75 |
| B | 97,2 | 1,77 |

| | | |
|---|---|---|
| ¹) 20%ige Lösung in Toluol bei 25°C nach DGF-M-III 8(75) | | |

Die Beispiele zeigen, daß auf den für das Abwasser und somit für die Umwelt nachteilig wirkenden Phasentransferkatalysator, der nach dem Stand der Technik als notwendig erachtet wird, vorteilhaft verzichtet werden kann. Dies wird erreicht durch die Verwendung eines wiederverwendbaren Lösemittelgemisches. Überraschenderweise läuft die Umsetzung der Edukte dadurch vollständiger ab als bei der Verwendung eines Phasentransferkatalysators. Man erhält höhere Polymerisationsgrade - erkennbar an der höheren Lösungsviskosität der Polymerisationsprodukte - unter gleichen Polymerisationsbedingungen. Aus der Literatur wäre zu erwarten gewesen, daß Phasentransferkatalysatoren bei Mehrphasenreaktionen wie der im vorliegenden Fall vorteilhafter seien.

## Patentansprüche

1. Verfahren zur Herstellung von Polyalkyl-1-oxa-diazaspirodecan-Verbindungen der Formel I 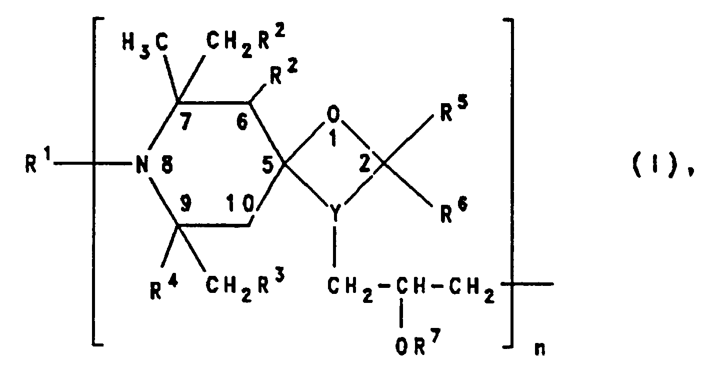 worin
n eine ganze Zahl von 1 bis 50 ist und
Y die Bedeutung einer Gruppe der Formel II oder III 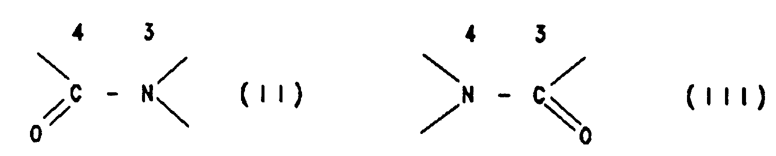 hat, wobei die Indices 3 bzw. 4 die Ringpositionen im Diazaspirodecansystem angeben und eine Bindung des Stickstoffs mit einer CH₂-Gruppe der Propylen-2-oxygruppe verknüpft ist,
R¹ ein Wasserstoffatom, ein Sauerstoffatom, eine NO-Gruppe, eine C₁-C₁₂--Alkylgruppe, eine Allylgruppe, eine C₁-C₂₂-Acylgruppe, eine Benzylgruppe, eine C₁-C₁₂-Alkyloxy-Gruppe oder eine C₃-C₁₂-Cycloalkoxy-Gruppe,
R² und R³ entweder gleich sind und ein Wasserstoffatom oder eine C₁-C₅-Alkylgruppe bedeuten, wobei dann R⁴ eine Methylgruppe ist, oder
R² ein Wasserstoffatom oder eine C₁-C₅-Alkylgruppe ist und R³ und R⁴ zusammen mit den sie verbindenden Kohlenstoffatomen eine C₅- oder C₆-Cycloalkylgruppe oder eine Gruppe der Formel 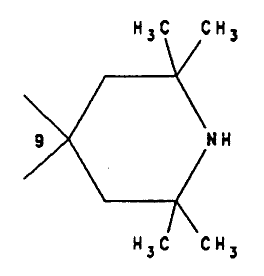 bilden,
R⁵ und R⁶ gleich oder verschieden sind und für ein Wasserstoffatom, eine C₁-C₃₀-,-Alkylgruppe, für eine unsubstituierte oder durch Chlor oder C₁-C₄-Alkyl substituierte C₇-C₁₂-Phenylalkylgruppe stehen, oder
R⁵ und R⁶ zusammen mit dem sie verbindenden Kohlenstoffatom eine unsubstituierte oder durch bis zu vier C₁-C₄-Alkylgruppen substituierte C₅-C₁₈-Cycloalkylgruppe oder eine Gruppe der Formel 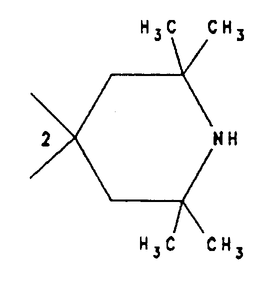 bilden,
R⁷ bei n = 1 keine Bedeutung hat, sodaß das Sauerstoffatom mit der endständigen CH₂-Gruppe verbunden ist und einen Oxiranring bildet,
R⁷ bei n > 1 ein Wasserstoffatom oder eine C₁-C₂₂-Acylgruppe ist, oder in der endständigen Monomereneinheit keine Bedeutung hat, so daß das Sauerstoffatom mit der enständigen CH₂-Gruppe verbunden ist und einen Oxiranring bildet, durch Umsetzung einer Verbindung der Formel IV 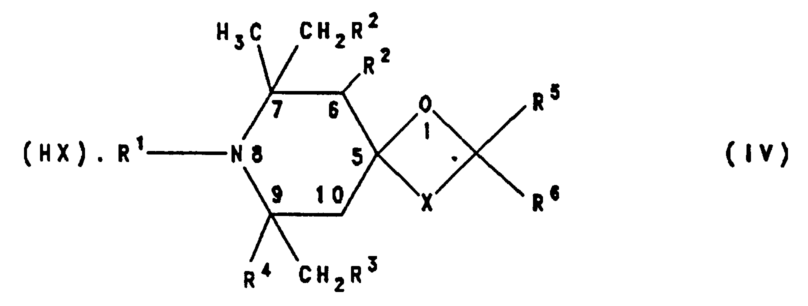 worin R¹, R², R³, R⁴, R⁵ und R⁶ die obengenannte Bedeutung haben und (HX) ein Säurerest ist, oder deren Salz mit einer Protonensäure mit einem Epihalohydrin der Formel V 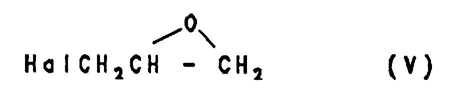 worin Hal ein Chlor-, Brom- oder Jodatom ist, im Molverhältnis 1:1 bis 1:10 in Anwesenheit eines Alkalihydroxyds in einem organischen Lösemittel und, bei n > 1, Erhitzen der entstandenen Epoxiverbindung VI 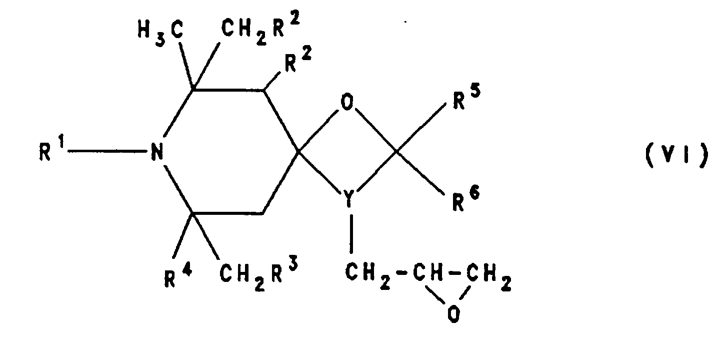 worin R¹, R², R³, R⁴, R⁵ und R⁶ die obengenannte Bedeutung haben, auf eine Temperatur von 100 bis 240°C, dadurch gekennzeichnet, daß die Umsetzung der Verbindung der Formel IV mit der Verbindung der Formel V in Gegenwart der äquimolaren bis zwanzigfach molaren Menge festen Alkalimetallhydroxids oder der entsprechenden Menge einer Mischung aus festem Alkalimetallhydroxid und Wasser im Gewichtsverhältnis 1:9 bis 9:1 als alleinigem Katalysator in einem Lösemittelgemisch aus mindestens einem C₁-C₄-Alkohol, ausgenommen Diethylenglykol und gegebenenfalls einem inerten organischen Lösemittel durchgeführt wird.

2. Verfahren nach Anspruch 1, dadurch gekennzeichnet, daß der Alkohol Isopropanol ist.

3. Verfahren gemäß Anspruch 1, dadurch gekennzeichnet, daß das verwendete inerte Lösemittel Toluol oder Xylol ist.

4. Verfahren gemäß Anspruch 1, dadurch gekennzeichnet, daß als Katalysator Natriumhydroxid in fester Form oder im Gemisch mit Wasser verwendet wird.

5. Verfahren gemäß Anspruch 1, dadurch gekennzeichnet, daß die Verbindung der Formel IV 2,2,4,4-Tetramethyl-7-oxa-3,20-diaza-21-oxo-dispiro-[5,1,11,2]-heneicosan oder dessen Hydrochlorid ist.

6. Verfahren gemäß Anspruch 1, dadurch gekennzeichnet, daß die Verbindung der Formel V Epichlorhydrin ist.

## Claims

1. A process for the preparation of a polyalkyl-1-oxadiazaspirodecane compound of the formula I 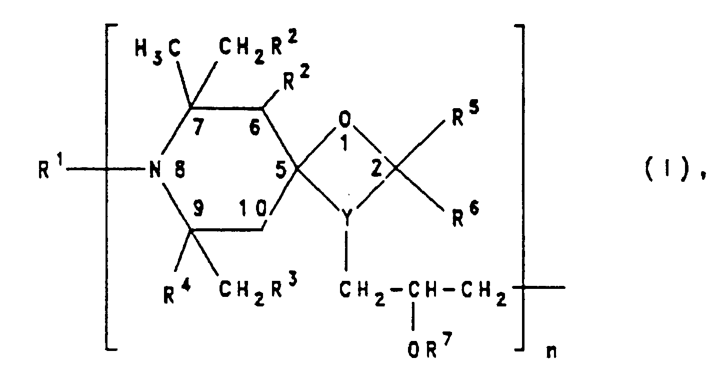 in which
n is an integer from 1 to 50 and
Y is a group of the formula II or III 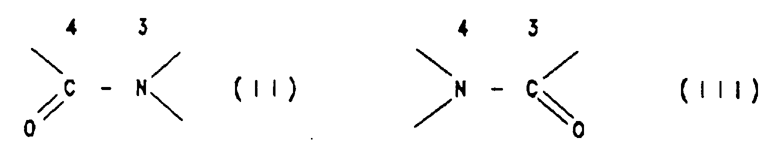 in which the indices 3 and 4 indicate the ring positions in the diazaspirodecane system and one bond of the nitrogen is linked to a CH₂ group of the propylene-2-oxy group,
R¹ is a hydrogen atom, an oxygen atom, an NO group, a C₁-C₁₂-alkyl group, an allyl group, a C₁-C₂₂-acyl group, a benzyl group, a C₁-C₁₂-alkyloxy group or a C₃-C₁₂-cycloalkoxy group,
R² and R³ are either identical and are a hydrogen atom or a C₁-C₅-alkyl group, in which case R⁴ is a methyl group, or
R² is a hydrogen atom or a C₁-C₅-alkyl group and R³ and R⁴, together with the carbon atoms joining them, form a C₅- or C₆-cycloalkyl group or a group of the formula 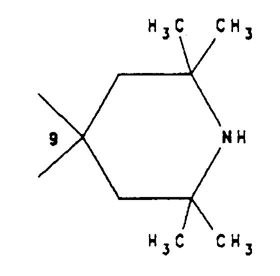
R⁵ and R⁶ are identical or different and represent a hydrogen atom, a C₁-C₃₀-alkyl group or a C₇-C₁₂-phenylalkyl group which is unsubstituted or substituted by chlorine or C₁-C₄-alkyl,or
R⁵ and R⁶, together with the carbon atom joining them, form a C₅-C₁₈-cycloalkyl group which is unsubstituted or substituted by up to four C₁-C₄-alkyl groups, or a group of the formula 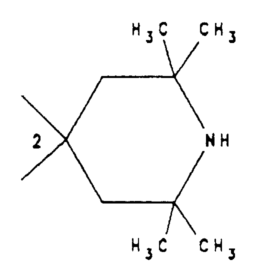
R⁷, if n = 1, has no meaning, so that the oxygen atom is bonded to the terminal CH₂ group and forms an oxirane ring, or
R⁷, if n > 1, is a hydrogen atom or a C₁-C₂₂-acyl group, or has no meaning in the terminal monomer unit, so that the oxygen atom is bonded to the terminal CH₂ group and forms an oxirane ring,
by reaction of a compound of the formula IV 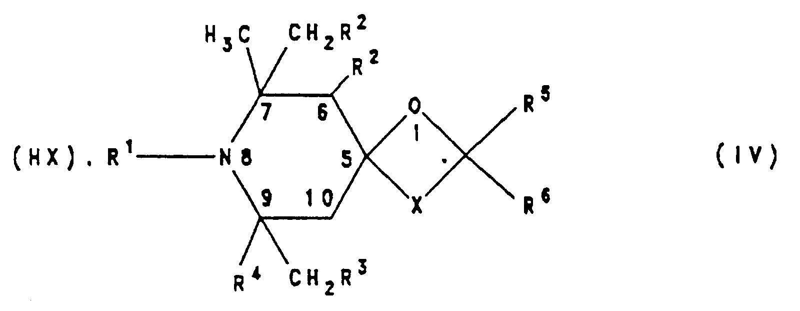 in which R¹, R², R³, R⁴, R⁵ and R⁶ have the above-mentioned meaning and (HX) is an acid radical, or a salt thereof with a proton acid, with an epihalohydrin of the formula V 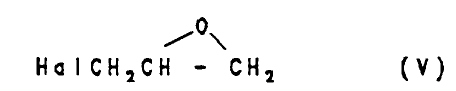 in which Hal is a chlorine, bromine or iodine atom, in a molar ratio of 1:1 to 1:10 in the presence of an alkali metal hydroxide in an organic solvent and, if n > 1, heating the resulting epoxy compound VI 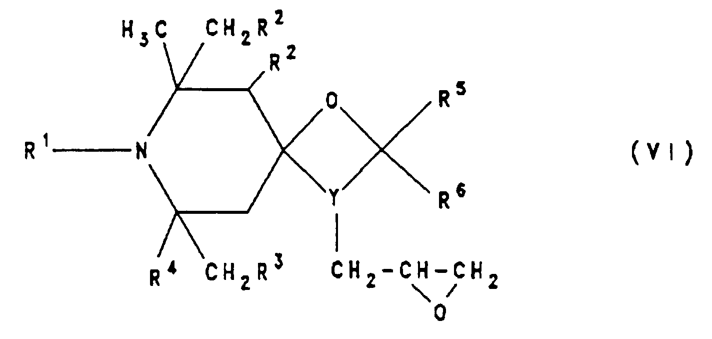 in which R¹, R², R³, R⁴, R⁵ and R⁶ have the above-mentioned meaning, to a temperature of 100 to 240°C, which comprises carrying out the reaction of the compound of the formula IV with the compound of the formula V in the presence of the equimolar to twenty times the molar amount of solid alkali metal hydroxide or of the corresponding amount of a mixture of solid alkali metal hydroxide and water in a weight ratio of 1:9 to 9:1 as the sole catalyst in a solvent mixture of at least one C₁-C₄ alcohol, with the exception of diethylene glycol, and if appropriate an inert organic solvent.

2. The process as claimed in claim 1, wherein the alcohol is isopropanol.

3. The process as claimed in claim 1, wherein the inert solvent used is toluene or xylene.

4. The process as claimed in claim 1, wherein sodium hydroxide in solid form or mixed with water is used as the catalyst.

5. The process as claimed in claim 1, wherein the compound of the formula IV is 2,2,4,4-tetramethyl-7-oxa-3,20-diaza-21-oxo-dispiro-[5,1,11,2]-heneicosane or its hydrochloride.

6. The process as claimed in claim 1, wherein the compound of the formula V is epichlorohydrin.

## Revendications

1. Procédé pour préparer des polyalkyl-1-oxadiazaspirodécanes de formule I 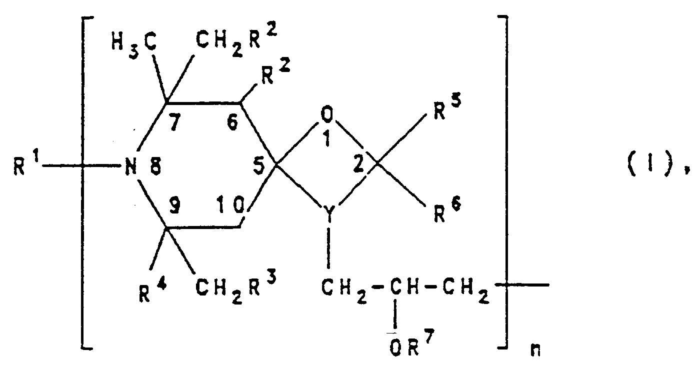 dans laquelle
n est un nombre entier de 1 à 50, et
Y représente un groupe de formule II ou III 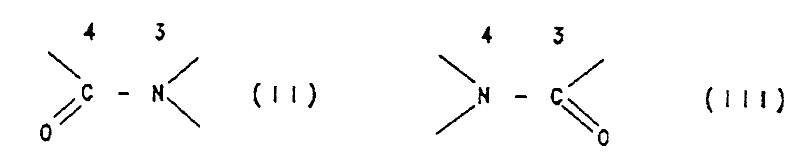 où les indices 3 et 4 représentent les positions dans le cycle diazaspirodécane et une liaison de l'azote est reliée à un groupe CH₂ du groupe propylène-2-oxy,
R¹ est un atome d'hydrogène, un atome d'oxygène, un groupe NO, un groupe alkyle en C₁-C₁₂, un groupe allyle, un groupe acyle en C₁-C₂₂, un groupe benzyle, un groupe alkyloxy en C₁-C₁₂ ou un groupe cycloalcoxy en C₃-C₁₂,
R² et R³ sont soit identiques et représentent chacun un atome d'hydrogène, soit un groupe alkyle en C₁-C₅, auquel cas R⁴ est alors un groupe méthyle, ou bien R² est un atome d'hydrogène ou un groupe alkyle en C₁-C₅, et R³ et R⁴, avec les atomes de carbone qui les relient, forment un groupe cycloalkyle en C₅ ou en C₆, ou encore un groupe de formule 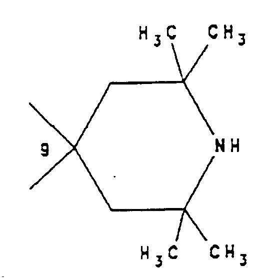
R⁵ et R⁶ sont identiques ou différents et représentent chacun un atome d'hydrogène, un groupe alkyle en C₁-C₃₀, Un groupe phénylalkyle en C₇-C₁₂ non-substitué ou substitué par des substituants chloro ou alkyle en C₁-C₄, ou bien
R⁵ et R⁶, avec l'atome de carbone qui les relie, forment un groupe cycloalkyle en C₅-C₁₈ non-substitué ou substitué par jusqu'à quatre groupes alkyle en C₁-C₄, ou encore un groupe de formule 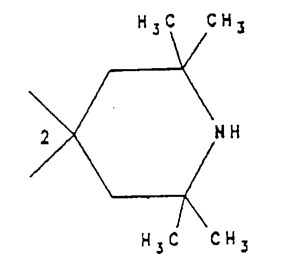
R⁷, quand n = 1, n'a pas de signification, de sorte que l'atome d'oxygène est relié au groupe CH₂ terminal et forme un noyau oxiranne,
R⁷, quand n > 1, est un atome d'hydrogène ou un groupe acyle en C₁-C₂₂, ou n'a pas de signification dans l'unité monomère terminale, de sorte que l'atome d'oxygène est relié au groupe CH₂ terminal et forme un groupe oxiranne,
par réaction d'un composé de formule IV 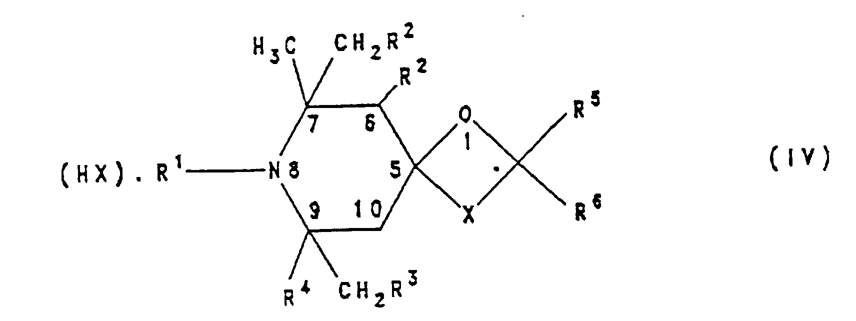 dans laquelle R¹, R², R³, R⁴, R⁵ et R⁶ ont les significations données ci-dessus, et (HX) est un résidu d'acide, ou leur sel avec un acide protonique, avec une épihalogènhydrine de formule V 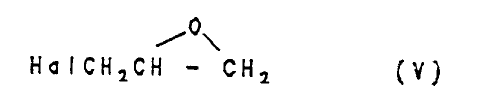 dans laquelle Hal est un atome de chlore, de brome ou d'iode, selon un rapport en moles de 1:1 à 1:10, en présence d'un hydroxyde d'un métal alcalin dans un solvant organique, et, pour n > 1, chauffage du composé époxyde VI obtenu 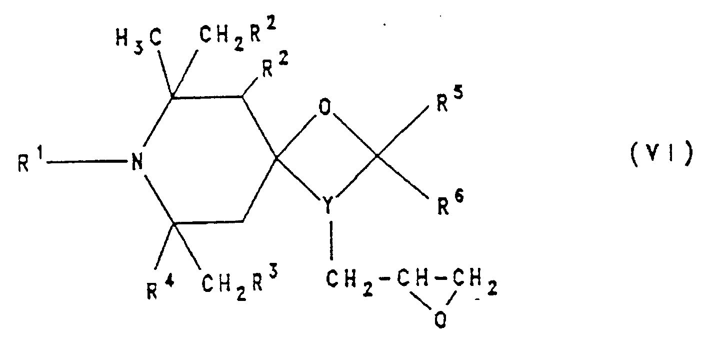 dans laquelle R¹, R², R³, R⁴, R⁵ et R⁶ ont les significations données ci-dessus, à une température de 100 à 240°C, caractérisé en ce que la réaction du composé de formule IV avec le composé de formule V est mis en oeuvre en présence d'une quantité comprise entre une quantité équimolaire et vingt fois molaire d'un hydroxyde solide d'un métal alcalin ou de la quantité correspondante d'un mélange d'un hydroxyde solide d'un métal alcalin et d'eau, selon un rapport pondéral de 1:9 à 9:1, servant de catalyseur unique dans un mélange de solvants constitué d'un alcool en C₁-C₄, à l'exception du diéthylèneglycol, et éventuellement d'un solvant organique inerte.

2. Procédé selon la revendication 1, caractérisé en ce que l'alcool est l'isopropanol.

3. Procédé selon la revendication 1, caractérisé en ce que le solvant inerte utilisé est le toluène ou le xylène.

4. Procédé selon la revendication 1, caractérisé en ce qu'on utilise en tant que catalyseur de l'hydroxyde de sodium sous forme solide ou en mélange avec de l'eau.

5. Procédé selon la revendication 1, caractérisé en ce que le composé de formule IV est le 2,2,4,4-tétraméthyl-7-oxa-3,20-diaza-21-oxo-dispiro[5,1,11,2]-hénéicosane ou son chlorhydrate.

6. Procédé selon la revendication 1, caractérisé en ce que le composé de formule V est l'épichlorhydrine.
